**Europäisches Patentamt**

**European Patent Office**

**Office européen des brevets**

(19)

(11) **EP 1 627 686 A1**

(12) **EUROPEAN PATENT APPLICATION**

(43) Date of publication:
**22.02.2006 Bulletin 2006/08**

(51) Int Cl.:
*B01L 3/00* (2006.01)    *G01N 30/92* (2006.01)
*G01N 30/38* (2006.01)

(21) Application number: **05024473.0**

(22) Date of filing: **11.04.2002**

(84) Designated Contracting States:
**AT BE CH CY DE DK ES FI FR GB GR IE IT LI LU MC NL PT SE TR**
Designated Extension States:
**AL**

(30) Priority: **26.04.2001  EP 01870091**

(62) Document number(s) of the earlier application(s) in accordance with Art. 76 EPC:
**02732596.8 / 1 383 603**

(71) Applicant: **VRIJE UNIVERSITEIT BRUSSEL**
**1050 Brussel (BE)**

(72) Inventors:
• **Desmet, Gert**
**1982 Elewijt (BE)**
• **Baron, Gino**
**3080 Tervuren (BE)**

(74) Representative: **Brants, Johan P.E. et al**
**De Clercq, Brants & Partners cv**
**Edgard Gevaertdreef 10a**
**9830 Sint-Martens-Latem (BE)**

Remarks:
This application was filed on 09 - 11 - 2005 as a divisional application to the application mentioned under INID code 62.

(54) **Method for acceleration and intensification of target-receptor binding and device therefor**

(57)    The present invention relates to a device for the simultaneous conduction of a multiplicity of binding reactions on a substrate with a first surface, said surface comprising discrete and isolated locations of one such binding reaction, said binding reaction being generated upon contact between the substrate and a fluidic sample comprising one or more target molecules capable of binding with at least one receptor molecule at least partly tethered to said first surface of the substrate, and further comprising means for enhancing the diffusion fluidic flow of the sample over the substrate, whereby said means for enhancing the diffusion fluidic flow comprises shear-force field generating means, whereby said means comprise a second surface positioned in a flow interacting manner in said flow channel, said second surface is able to be moved in a substantial parallel mode over the first surface.

The invention further relates to a method for binding one or more target molecules comprised within a fluid sample to at least one receptor molecule at least partly tethered to a first surface in a flow channel, wherein said target molecules are transported parallel to said first surface by means of shear-force field generating means, wherein the shear-force field generating means comprises a second surface, which second surface is moved in a substantially parallel mode past or over said first surface.

**EP 1 627 686 A1**

**Description**

FIELD OF THE INVENTION

The present invention relates to a novel operation method for biological and chemical analysis systems relying on target-receptor binding events.

BACKGROUND

With the increased demand for genomic and proteomic information from the pharmaceutical and the agro-biotechnology industry and from the medical sector (cfr. clinical diagnostics), the past decade has witnessed a tremendous interest in the development of miniaturised screening or binding assay systems. This trend towards miniaturised and on-chip analysis systems has been initiated by the introduction of the powerful micro-machining techniques originally developed for the micro-electronics industry into the field of analytical and bioanalytical chemistry. The most important advantages of miniaturization are: the enhanced mass transfer kinetics originating from the decreased mass transfer distances, the possibility to perform multiple analysis in parallel on a small surface, and the reduction of the sample and reagent consumption. As a result, a higher throughput (and hence more information per unit time) and better over-all process economics are obtained.

Currently, two main formats for miniaturized biochemical analysis systems are being adopted. In one format, commonly referred to as micro-array systems and being the logic evolutionary step of the 96-well micro-titer plate systems used in so many biochemical assay and screening procedures, reagents or unknown samples are brought into contact with a large set of different, but known reagent or receptor molecules immobilized at the surface of a fixed carrier plate and arranged in arrays of (typically) circular spots. In the second format, commonly referred to as microfluidics, fluid is pumped around through a manifold of etched micro-channels to perform separations, mixing operations and to deliver sample at predefined locations, all on the same miniscule surface of a conventional size micro-electronics chip.

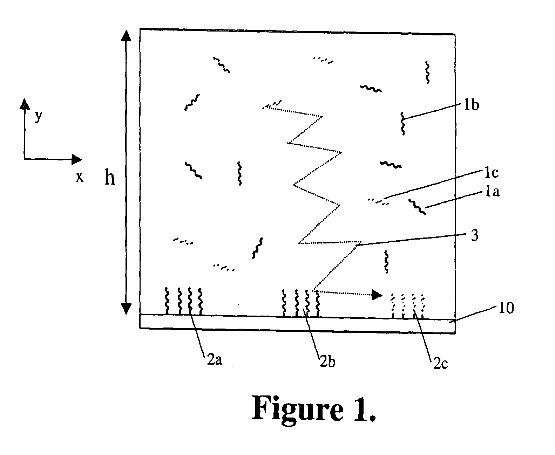

Traditionally, DNA or protein micro-array screening assays are effectuated by applying a drop of sample between two parallel plates, one carrying a high density micro-array of so-called probe or receptor molecules spots, while the other plate simply serves to seal off the system and to prevent evaporation of the sample liquid. In such systems, the transport of the sample molecules towards the receptor spots often occurs in a passive mode, i.e., the transport still has to occur by pure molecular diffusion (cfr. diffusive path 3 in Fig. 1). As a result, micro-array screenings are still relatively slow, and usually have to occur overnight.

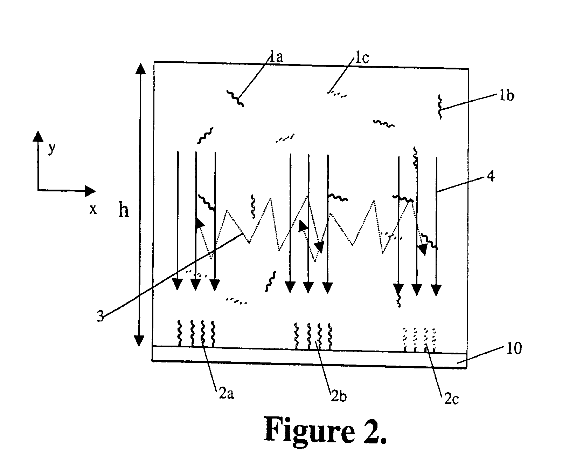

Two recent disclosures have been proposed to alleviate the micro-array diffusion problem. In US Patent 6,017,696 the transport in the y-direction (i.e., perpendicular to the surface carrying the probe spots, as indicated in the present application by arrows 4 in Fig. 2) is enhanced by applying an electrical field perpendicular to the surface carrying the probe spots (US Patent 6,017,696). In an another approach, a nano-porous membrane is used through which a filtration flow (i.e., also in the y-direction as indicated by arrows 4 in Fig. 2) is directed (US patent 5,843,767). A constrain of both concepts is that the transport is only accelerated in the y-direction. This implies that, during the hybridization process, the immobilized probe or receptor molecules present in a given spot are only contacted with the target molecules present in the fluid volume situated directly above the given spot. Very often, the number of matching molecules present in this limited volume is not sufficient to generate a measurable signal. In both methods cited above, an additional supply of matching molecules coming from the fluid volumes covering the neighboring spots still has to occur via the (slow) diffusion process (following the diffusive path 3 indicated in present Fig. 2). Another drawback of the method in US Patent 6,017,696 is that the electrical field may induce undesirable non-specific bindings, such that an additional stringency test step has to be added. Furthermore, the use of an electrical field to accelerate the radial mass-transfer is not applicable for target molecules having a net zero charge. Another drawback is that a number of specific problems, such as the formation of gas bubbles at the electrodes, has to be coped with. For the nano-porous filter method in US patent 5,843,767, an additional drawback can be found in the fact that the generation of the flow through the nano-pores requires the application of a sufficiently large excess pressure near one side of the membrane. This inevitably brings along sealing problems. Furthermore, as the flow rate through the pores is proportional to the applicable excess pressure, the achievable flow rate through the device has a clear upper limit (cfr. Poiseuille's law).

Apart from finding an answer to the question how much matching target molecules are present in a given sample, other applications, for example in drug screening, also require information on the rate of the binding process (for example to investigate competitive binding phenomena). Currently, the most frequently used system for the measurement of target-receptor binding kinetics is the so-called BIAcore apparatus (commercialized by Biacore AB, Upsalla, Sweden), in which samples are flown past a spot of known receptor molecules and wherein the amount bound is continuously monitored by means of a surface detection technique such as surface plasmon resonance. The Biacore system suffers from an important drawback. As the system is pressure-driven, relatively large flow cuvettes are needed. As a consequence, the diffusion time needed to reach the receptor molecules is part of the measured binding time. The obtained kinetic measurements hence do not exclusively relate to the intrinsic binding process, but they are biased by the slow diffusion process. This problem is in fact common to all the binding kinetics measurement systems known in the art. To obtain more accurate measurement of the intrinsic binding process itself, it would hence be beneficial to have a method which is able to substantially accelerate the diffusional transport step. In addition, it would be preferable to have an acceleration method which does not rely on the application of an electrical field, because this would certainly influence the binding process itself.

Generating flows past spots of immobilized receptor molecules arranged on the surface of microfluidic channels is for example known in the art of miniaturized biosensor manufacturing. As is known to those skilled in the art of microfluidies, fluid motions in microfluidic channel systems are in general not generated by means of a pressure pump, but by means of an electrical force field. This is due to the large flow resistances and the correspondingly large pressure-drops encountered in pressure-driven micro-channel systems (cfr. Poiseuille's law). Electrically-driven flows (also referred to as electro-osmotic flows, EOF) do not suffer from the pressure drop problem, and are hence much better suited for microfluidic applications. The problem of electrically-driven flows however is that the they are inevitably accompanied by electro-migratory effects (in both the axial and radial direction). For some applications, such as Capillary Electrophoresis, this is a highly desired effect, but for the transport of a group of different sample molecules at a predefined velocity, these electro-migratory effects might lead to an undesirable axial dispersion or might lead to undesirable wall adsorption or wall repulsion, depending on the charge of the sample molecules (in EOF, the walls always carry a net charge). Another drawback of electrically-driven flows is that, as their generation relies on the formation of an electrical double layer along the walls of the channel, their flow rate is easily disturbed (and hence suffers from poor reproducibility) by adsorption of the sample molecules to the channel walls. This problem is especially severe in the case of large and charged molecules such as proteins. Yet another drawback is that, when the capillaries or the channels become too narrow, the double layers of the opposing channel walls start to overlap, such that radial electrical attraction and repulsion effects become dominant over the desired axial movement. This prevents the use of sub-micron thin channels. Finally, electrically-driven flows are (similarly to pressure-driven flows) also subjected to the existence of an upper velocity limit. As the fluid velocity is directly proportional to the applied electrical voltage drop, and as there is a clear upper limit (about 30 kV) for this voltage drop, for safety reasons and to avoid excessive Joule heating and bubble formation, the existence of an upper velocity limit is obvious.

In WO 99/61896, a device is disclosed wherein the binding of biomolecules is enhanced by creating thin fluid layers by forcing air bubbles through a flow-through detection cuvette. The advantageous effect, i.e., the creation of a thin fluid layer with ultra-short diffusion times, of these air bubbles is however only temporarily. Furthermore, the entire cuvette needs to be filled with sample, such that the device still requires sample volumes, which are at least of the order of the bubble size volume. When the bubbles are past through the cuvette, the major fraction of the sample will run ahead of the bubble and will hence be pushed out of the cuvette without having reached the receptor molecules attached to the wall.

The object of the present invention is to provide improved devices and methods which result in shortened screening or assay times, improved screening detection limits, and more accurate measurements of binding kinetics and equilibria. In particular, said device and method of the current invention nearly completely eliminates the rate-limiting diffusion-step.

In the present invention, a device and a method are disclosed wherein the entire device is miniaturized and wherein a thin fluid layer is continuously maintained by keeping the fluid between two substantially parallel solid surfaces. By moving one of the two surfaces to generate a continuous shear-flow, we ensure that each molecule of the sample passes the receptor molecules within a distance, which is sufficiently small to be elapsed by pure molecular diffusion during the dwell-time.

<u>SUMMARY</u>

It is the aim of the present invention to provide a novel and generally applicable operation mode for the micro-array and bio-sensor systems currently used for, but not limited to, the detection and the quantitation of biological molecules such as nucleic acids and proteins (including hormones, hormone receptors, antibodies, antigens, structural proteins, enzymes, etc...), the screening of the therapeutic effect of molecules, enzymatic reaction assays, the purification of production quantities of valuable bio- and therapeutic molecules, and the measurement of ligand/receptor binding affinity and kinetics, overcoming the above drawbacks and yielding superior analysis speeds and detection limits and allowing more accurate binding kinetics measurements.

The present invention may be explained by a theoretical calculation (represented in full detail in the Description Section) showing that, instead of contacting contact a receptor molecule spot directly with a sample fluid layer of say 100 micron thick, it is more advantageous to contact the spot with a thin fluid layer of say 0.1 micron thick and to continuously refresh the layer a 1000 times, provided that the refresh rate can be made sufficiently large. As they are based on the dragging action of a moving surface, inducing a fluid velocity equal to one half of the moving surface velocity, independent of channel thickness or channel length, shear-force flows are the single flow type able to produce sufficiently large fluid velocities through sub-micron thin channels.

According to a first aspect, the present invention relates to a device for the conduction of target-receptor binding reactions within a flow channel provided with receptor molecules, whereby said binding reaction is being generated upon contact of a fluidic sample comprising one or more target molecules with said receptor molecules, said device further comprises:

- a first surface provided with discrete and isolated locations for such a binding reaction (spots) within said flow channel, and
- means for enhancing the diffusion fluidic flow of the sample in said flow channel, whereby said means for enhancing the diffusion fluidic flow consisting essentially of a second surface positioned in a flow interacting manner in said flow channel, said second surface is able to be moved in a substantial parallel mode over the first surface.

These discrete and isolated locations of one such binding reaction are also commonly defined as spots. Typical spot sizes vary from 50-200 $\mu$m.

According to another embodiment said said second surface is larger than said first surface.

According to yet another embodiment said second surface is a linearly movable flat plate, a linearly movable flexible belt, a rotatable device or a rotatable cilinder.

In another aspect, the present invention also relates to a method for binding one or more target molecules comprised within a fluid sample to at least one receptor molecule at least partly tethered to a first surface of a substrate, wherein said target molecules are transported parallel to said first surface by means of a second surface, which second surface is moved in a substantially parallel mode past or over said first surface.

Other more preferred embodiments as described in full detail in the description below.

Within the meaning of the present invention a "first surface" denotes in general a surface or a substrate provided with one or more different or the same receptor molecules. These receptor molecules are generally at least partly tethered to said surface. Within the meaning of the present invention a "second surface" denotes a surface which is in a movable manner provided in the flow channel. Movement of said second surface will provide for a shear-force field acting upon a fluidic sample, if present, in said flow channel.

In several embodiments, the first and second surface delimit the flow channel. As in these embodiments, said first and second surface can be moved independently from each other. As a consequence the flow channel according to these embodiments are characterized in that they are not hermetically sealed along their mantle surface, but simply consist of two independent surfaces.

The target molecules are in general present in a fluidic sample forced over these receptor molecules in order to enhance the binding efficacy.

The method and devices according to the present invention rely on the combination of three specific features of shear-driven micro-channel flows. The first feature is that the channel thickness may be selected such that it is only a minimal number of times (preferably 1.5 to 5 times larger) larger than the dimensions of the molecules to be analysed. In this way, target molecules may be convectively transported to within a few molecular diameters of matching receptor molecules immobilized on a solid surface, hence nearly completely eliminating the time needed for diffusion. The second feature is that the flow velocity may be made large enough to transport sample volumes of the order of 10 to 200 $\mu$l through micro-array channels with a volume of the order of only 0.1 $\mu$l while, in combination with the first feature, still gaining substantially in screening time with respect to the conventional diffusion-based DNA and protein micro-arrays wherein the entire 10 to 200 $\mu$l sample volume is contacted directly with the entire array. The third feature is that, because of the fact that the degree of axial dispersion in channels with a sub-micron thickness is extremely small, short sample plugs (i.e., with a length of the order of 10 to 500 $\mu$m) may be injected at a high frequency and may subsequently be transported through a microfluidic channel without any substantial intermixing between the successive sample plugs. This third feature allows, in combination with the first feature, true high-throughput screening analysis of, for example, potential therapeutic molecules. The small degree of axial dispersion also implies that minute amounts of sample may be analyzed and recovered with a minimum of dilution. This is a highly desired characteristic in bioanalytical and purification applications. As in a shear-driven flow the fluid velocity is independent of the nature (charge, size, etc...) of the fluid substances to be transported, it is yet another advantage of the method according to the present invention that molecules of a different nature may be transported in a single plug, i.e. at the same velocity. This is especially advantageous to investigate binding or reaction events involving a co-factor, or to perform competitive binding assays. An additional advantage with respect to electrically driven flows, where the velocity profile is uniform, is that the velocity of shear-force flows varies linearly over the channel thickness and is nearly zero the stationary surface where the binding has to occur. The binding can hence occur with a minimal influence of the applied shear-force. In an electrically-driven system, the electrical field is equally strong everywhere, such that it has a larger possibility of disturbing the binding process.

For the selective binding of long linear molecules (nucleic acids, denatured proteins), it is yet another advantage of the method according to the present invention that the linear velocity field of shear-force driven flows induces an advantageous pre-orientation of the molecules. Because of the large shear-forces, linear molecules will be stretched along the direction of the flow field, and will hence be less coiled. In this way, an enhanced target-receptor contact can be obtained.

It is yet another advantage of the method according to the present invention that the fluid velocity can be used as an additional stringency control parameter. Non-matching, and hence more loosely bound molecules will detach to a larger extent than perfect matching molecules under the influence of a strong shear-force field.

It is yet another advantage of the embodiments according to the present invention that it is very straightforward to switch from a continuous flow mode to a transport mode consisting of a continuous series of flow-stop sequences. This possibility is highly advantageous in cases where a strong convective flow disturbs the binding events: during the (short) stop periods, the binding process can take place undisturbed by any convection effects, while the flow periods are used to generate a rapid renewal of the fluid layer covering a given receptor molecule spot.

BRIEF DESCRIPTION OF THE DRAWINGS

Figure 1. Schematic representation of the diffusive transport in conventional diffusion-based DNA and protein screening devices.

Figure 2. Schematic representation of prior art solutions for the acceleration of the radial transport, involving flow-through porous membranes or electronically assisted hybridization.

Figure 3. Schematic representation of the flow organization in the shear-driven DNA and protein screening devices according to the present invention.

Figure 4. Schematic representation of basic fluid transportation channels used in the presently disclosed target-receptor screening method: longitudinal view (a), cross-sectional view (b) and top view (c).

Figure 5. Fluid volume on top of probe spot in conventional diffusion-driven system (a) and in a shear-driven system according to the present invention (b).

Figure 6. Schematic representation of moving belt (a) and rotating disk devices according to the present invention: side view (b) and top view (c).

Figure 7. Top view of rotating disk device showing one of the numerous possibilities of the method according to the present invention to use one single moving surface to generate a flow past a multitude of receptor molecule surfaces.

Figure 8. Top view (a) and longitudinal view (b) of moving surface system carrying an array of micro-machined protrusions to provide additional sustainment of the fluid motion and/or to induce additional mixing.

Figure 9. Examples of arrays of micro-machined protrusions allowing to generate intermittent diversions of the flow direction to promote mixing in the z-direction.

Figure 10. Side view of position and conceptual design for sample reservoir device integrated with screening array surface.

Figure 11. Top view of shear-driven screening method in which the convective transport is only in one direction (a). Top view of second passage of sample after having turned the spot array surface by 90° (b).

Figure 12. Top view of shear-driven screening method in which a convective transport is established in two, mutually perpendicular directions.

Figure 13. Top view of shear-driven screening method in which both a translational and a rotational convective transport are established.

Figure 14. Schematic representation of dual rotating disk devices according to the present invention: top view of receptor surface (a), and side view of assembled apparatus with variable disk spacing, and showing the peripherical outflow of the sample fluid (b).

Figure 15. Schematic representation of dual rotating disk devices with variable disk spacing, and with a receptor surface having pores or micro-machined holes allowing the outflow of the sample fluid.

Figure 16. Schematic view of a method for the injection of sharply delimited sample plugs in the devices according to the present invention.

Figure 17. Schematic view of a high throughput transport of different sample plugs.

Figure 18. Schematic view of the different sequences involved in a method for the measurement of target-receptor binding kinetics according to the present invention, comprising shear-driven transport of sample molecules towards a binding section containing selective receptor molecules (a), contacting said sample molecules with said selective receptor molecules during a given time $t_{cont}$ (b), and transporting away the non-bound molecules (c).

Figure 19. Longitudinal view of an embodiment according to the present invention wherein the receptor molecules are immobilized in a recessed portion of the stationary surface.

DESCRIPTION

In a first aspect, the present invention relates to a device for the simultaneous conduction of a multiplicity of binding reactions on a substrate with a first surface, said surface comprising discrete and isolated locations of one such binding reaction, said binding reaction being generated upon contact between the substrate and a fluidic sample comprising one or more target molecules capable of binding with at least one receptor molecule at least partly tethered to said first surface of the substrate, and further comprising means for enhancing the diffusion fluidic flow of the sample over the substrate, characterized in that said means for enhancing the diffusion fluidic flow comprises a shear-force field generating means.

In particular, the device of the present invention allows to improve the performance of analytical diagnostic and screening systems based on the specific binding between ligand or target molecules in a sample with complementary receptor molecules tethered at a solid surface, and used for the detection, the quantitation and the functional analysis of biological and chemical analytes, including, but not limited to, nucleic acids, proteins, antibodies, antigens, hormones, hormone receptors, enzymes, and small molecules from combinatorial chemistry libraries. The apparatus of the invention may also be exploited to investigate the reaction rate and the product formation of enzymatic substrate conversions.

Referring to the drawings shown in the present document, it should be noted that, although the shape of some of the depicted molecules might suggest that the represented device and method of the invention as described further below only relate specifically to nucleic acids or antibodies, all the methods and devices according to the present invention relate to the entire group of chemical and biological analytes mentioned above.

Instead of being of a molecular nature, the receptor means can also be a micro-machined surface recession having a specific size and/or a shape allowing to selectively retain small crystals, whole cells or nano-devices.

Fig. 1 shows the fluid transport organization in conventional diffusion-based DNA and protein screening devices. In these devices, unknown target molecules 1 a, 1b, 1 c, etc... of different composition have to diffuse in both the y- and x-direction (i.e., they have to follow the tortuous diffusive path 3) before they can bind to one of the matching receptor molecule spots 2a, 2b, 2c, etc... immobilized at surface 10. Fig. 2 shows the fluid transport organization in the DNA screening devices according to the prior art in for example US patent 5843767 and US patent 6103479. In these devices, transport is only accelerated in the radial direction 4. During the rapid radial transport, the transport in the transversal direction 4, i.e., from spot to spot, still has to occur via the slow molecular diffusion following the diffusive path 3. As the time for the radial transport is orders of magnitude smaller than the time needed for the transversal spot-to-spot transport, the latter can not occur to a significant extent, and the detection limits remain low.

According to an embodiment of the present invention, the device of this invention as described above is characterized in that the shear-force field generating means comprises a second surface preferably being larger than said first surface, and able to be moved mechanically in a substantially parallel mode past said first surface.

In the present invention, the concept of shear-driven flows is used to accelerate target-receptor binding processes by exploiting the fact that in a shear-driven flow the channel thickness may be substantially reduced without having to sacrifice on the achievable fluid velocity. As is explained in a quantitiative manner further down below, the combination of both facts yields the key factor in the possibility to accelerate screening assays according to the present invention.

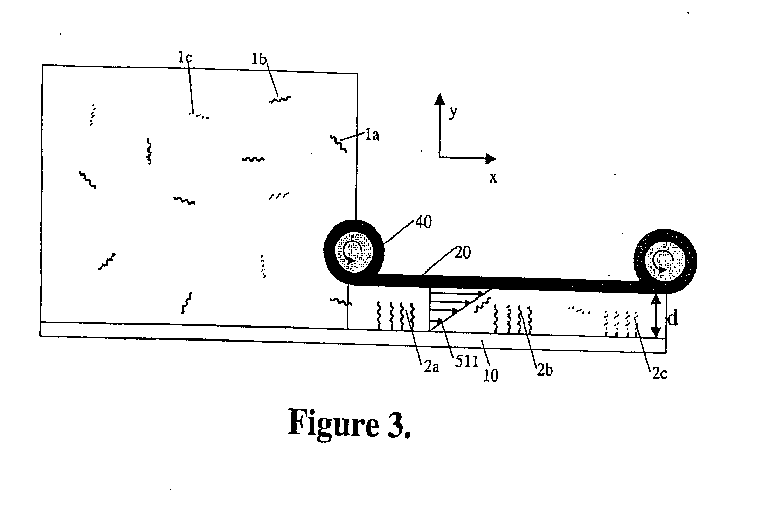

Fig. 3 shows a preferred embodiment of the fluid transport organization in the DNA and protein screening devices according to the present invention. The transport in the radial direction is herein accelerated by decreasing the fluid layer thickness, whereas in the transversal direction (i.e., the x-direction), a convective, and hence rapid spot-to-spot transport is organized. In a device according to the present invention, the sample containing the unknown target molecules 1a, 1b, 1c, etc... is transported in a very thin layer (with thickness d) past an array of spots of matching probe molecule spots 2a, 2b, 2c, etc... immobilized at a first surface 10, using a shear-force field. This shear-force field according to this embodiment is originating from the movement of a second surface 20, driven by any suitable motor system 40, and inducing a flow with a substantially linear profile 511. The second surface 20 constitutes together with the first surface 10 in this embodiment the boundaries of the flow channel.

Shear-driven forces are known as a transport force in separation techniques such as chromatography. In EP. Nr. 98/03485 for instance in the name of the present applicant, a number of possible channel designs allowing to establish a continuous shear-driven transport of mobile phase from an inlet to an outlet reservoir has been disclosed for an application in the field of liquid chromatography. It is disclosed how shear-driven flow may be established in, through and out a microfluidic channel consisting of two different wall parts, one part being substantially larger than the other part and being moved in a substantially parallel mode past the shorter part. This application is hereby incorporated by reference.

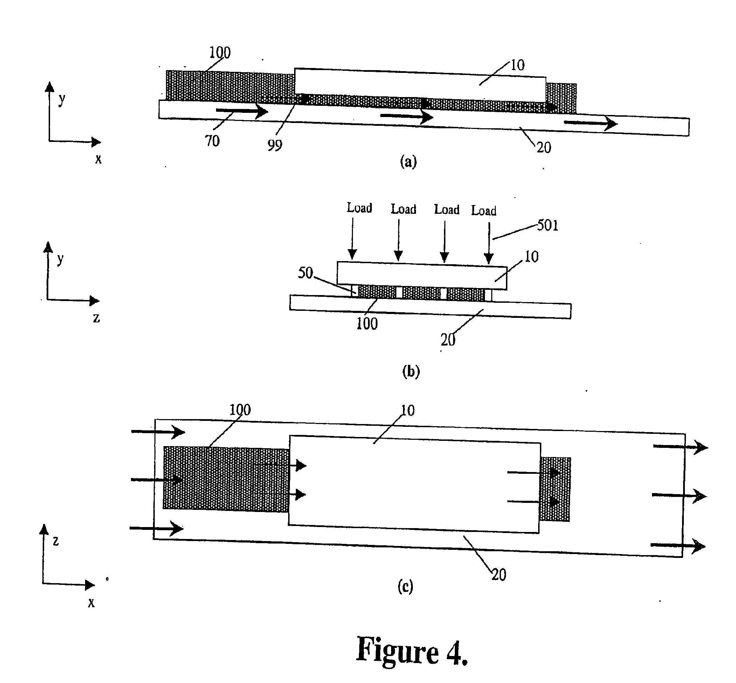

In figure 4a a basic fluid flow channel is shown in three views whereby a thin layer of sample 100 is transported along a micro-array chip surface 10 in the direction 99 by a shear-force effect originating from the moving wall part 20.

According to yet another embodiment, the device of the invention is further characterized in that the first surface comprises micro-machined and/or micro-structured surface recessions which preferably have approximately the same depth as the height of the receptor molecules immobilised thereon. This protects the receptor molecules from the high shear-field in the case of large fluid velocity applications.

Since channels are used which are only a minimal number of times (preferably between 1.5 and 5 times) larger than the target molecules to be analysed, the time needed for diffusion is decreased substantially and nearly completely eliminated. In this way, the radial transport is accelerated, whereas the transport in the lateral direction (i.e., from spot to spot) occurs by rapid convection, with flow velocities independent of the channel thickness.. In this way, the drawback of the small channel volume, which would normally lead to poor detection limits, may easily be overcome because of the continuous, rapid refreshment of the fluid layer inside the screening channel.

In the devices according to the present invention, the channel space formed by the moving second and the stationary first surface preferably has a flat-rectangular cross-section, i.e., the channels should preferably have a thickness between 10 nanometer and 10 micrometer, and more preferably between 50 and 1000 nanometer, while their width should be much larger, preferably between 100 micron and 10 cm (cfr. Fig. 4b, not to scale). The length of the channels should preferably be between 1 millimeter and 10 cm.

In a channel system as depicted in Figure 4, the flow (in direction 99) of the sample liquid 100 is generated by the drag or shear-force effect originating from the moving surface 20. In the present invention, advantage is taken from the fact that, as can be deduced from the linear flow profile 5 in Figure 3, the fluid velocity in a shear-driven flow is always equal to one half of the moving part velocity, independent of the length of the channel, the thickness of the channel or the nature of the fluid. Assuming for example a moving wall velocity of 10 cm/s, this implies that a thin layer of sample 100 (for example 50 nm thick) may be transported along a micro-array chip surface 10 of for example 5 cm long at a velocity of for example 5 cm/s. In a pressure-driven channel with a flat-rectangular cross-section this would require an impractically large inlet pressure of 60,000 bar. In the device according to the present invention, the above advantage of shear-driven flows is exploited to convectively transport molecular fluid substances to within a few molecular diameters of a (potentially) complementary receptor molecule of known composition and immobilized on a solid surface (cfr. Figure 3).

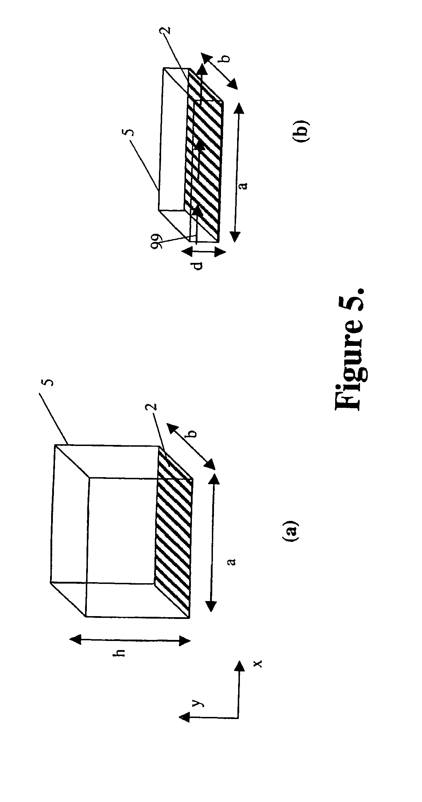

With reference to Figs. 3 and 5, the below reasoning allows to quantify the gain of the shear-driven screening method according to the present invention for the case of a DNA screening application. The reasoning starts by noting that typical DNA diffusion values (Chan et al., 1995) range between $D_{diff}=9.10^{-11}$ m²/s for 30-bp ssDNA, down to $D_{diff}=5.10^{-12}$ m²/s for 1000-bp ssDNA. Considering now the typical lay-out of a DNA-chip (cfr. the 1-dimensional representation given in Fig. 1), being a tiled array of circular spots with a typical diameter of 200 $\mu$m and with an intermediate spot distance of 30 to 50 $\mu$m, and considering that the time $\tau$ needed to elapse a given distance / can be estimated from Einstein's diffusion equation:

$$\tau = l^2/(2.D_{diff}), \qquad\qquad (1)$$

it can easily be verified that it takes about 1h for a DNA strand with $D_{diff}=1.10^{-11}$ m²/s to travel from one spot to another spot, and that it takes about $2.10^8$ seconds (200 million seconds) to travel the entire 7.5 cm length of a typical DNA micro-array (having the same dimensions as a conventional microscope slide). This basic calculation shows that, in a typical DNA-chip, a spot only 'sees' the fluid in its immediate neighbourhood. Calculating the radius from which a given spot is able to sample material during an overnight experiment of 15h, by solving eq. (1) for / and by putting $\tau=15$h, a sampling distance of the order of /=1mm is found. This implies that a given spot only 'sees' the sample located 1mm to its left and 1 mm to its right. This also explains why, in order to achieve a sufficient detection limit, relatively thick fluid layers, of the order of 20 to 100 micron have to be used in DNA microarrays (order of 100 $\mu$m, Duggan, D. J., *et al.*, 1999. *Nature Genetics Supplement 21,* 10-14).

In the shear-driven method according to the present invention (Fig. 3), in which the sample is smeared out in an ultra-thin fluid layer of for example 100 nm thick (or any other suitable thickness, depending on the dimensions of the DNA strands), eq. (1) predicts a 1 million-fold increase in radial diffusion rate (i.e., in the y-direction, see Figs. 1 and 3). This gain is extremely large, but it should be realised that a 100 nm thick layer also contains 1000 times less DNA target material. This implies that, in order to obtain comparable detection amounts, the layer has to be continuously refreshed. This is where one of the unique features of the shear-driven flow concept, i.e. the possibility to generate large fluid velocities, even in sub-micron channels, becomes especially useful. As the method according to the present invention allows to establish fluid velocities of the order of tens of centimeters per second, and even beyond that, the time needed for the transport across for example a 7.5 cm long DNA chip (which is of the order of 100 million seconds for a purely diffusion-driven system) may easily be reduced to a few seconds or tens of seconds (depending on the spot dwell time needed to let the molecules diffuse towards the receptor molecules and to complete the binding reaction).

Referring to Figure 5, and assuming a diffusion-based assay with a rectangular receptor molecule spot 2 (dimensions=a x b), and considering only radial diffusion (y-direction), the spot 'sees' a total liquid volume 5 of $V_a$=a.b.h. According to eq. (1), the time needed for total diffusion across the height h is:

$$\tau_{as} = h^2/(2.D_{diff}). \qquad\qquad (2)$$

Considering now the shear-driven approach, where the fluid volume 5 in contact with said receptor molecule spot 2 only has a height d, and where a convective action in direction 99 is used to transport the same volume $V_a$ across the spot, a decision has to be made on the applicable flow velocity. This value can be calculated from the fact the time $t_{dwell}$ during which a given fluid volume travels along the spot should be taken such that, within this dwell time $t_{dwell}$, the molecules have had the time to diffuse across the layer thickness d and have had the time to hybridise. Expressing the time needed for the hybridisation as being a given fraction or multitude of the diffusion time, we obtain, again according to eq. (1):

$$t_{dwell} = (1+\alpha).d^2/(2.D_{diff}), \qquad\qquad (3)$$

With this required residence time, the maximal allowable velocity v can be calculated as:

$$v=a/t_{res}, \qquad\qquad (4)$$

and with this velocity, the volumetric flow rate F in the shear-driven system may be calculated to be:

$$F=v.b.d=a\ b.d/t_{res} \qquad\qquad (5)$$

Taking typical values for d=0.1 $\mu$m and $D_{diff}$=1.10$^{-11}$ m$^2$/s and a=200 $\mu$m (typical spot size in micro-arrays) and assuming that $\alpha$=1, the required velocity according to eq. (4) is 0.2 m/s. Considering a 100 nm thick, and for example 5 cm long channel, it should be obvious that, considering the limitations of pressure-driven and electrically-driven flows, such a velocity can only be achieved in a shear-driven flow mode.

With the required dwell time $t_{dwell}$ given by eq. (3), it may easily be found that the total time $\tau_{SD}$ needed to let hybridise the total liquid volume $V_a$ is given by:

$$\tau_{SD}=V_a/F=(1+\alpha).h.d/(2.D_{diff}) \qquad\qquad (6)$$

Taking now the ratio of $\tau_{as}$ to $\tau_{SD}$:

$$\frac{\tau_{as}}{\tau_{SD}} = \frac{h}{d.(1+\alpha)}, \qquad\qquad (7)$$

and assuming that $t_{hybr}=t_{diff}$ (i.e., $\alpha$=1), eq. (7) shows that the gain which can be obtained by switching from a diffusion-based micro-array with height h=100 $\mu$m to a shear-driven micro-array with height d=0.1 $\mu$m is of the order of a factor 500.

The gain is even larger when, during a given screening, a given receptor molecule spot needs to 'see' an amount of liquid initially spread over N spots. With N sufficiently large (say N≥5), the characteristic diffusion distance in the diffusion-based array no longer corresponds to the radial height (l=h), but now has to be taken along the lateral coordinate, i.e., in the x-direction, such that l=N.a. With now $V_a$=N.a.b.h, and $\tau_{as}$= (N.a)$^2$/ (2.$D_{diff}$), and following the same reasoning as above, the ratio of $\tau_{as}$ to $\tau_{SD}$ becomes:

$$\frac{\tau_{as}}{\tau_{SD}} = \frac{N.a^2}{h.d.(1+\alpha)}, \qquad\qquad (8)$$

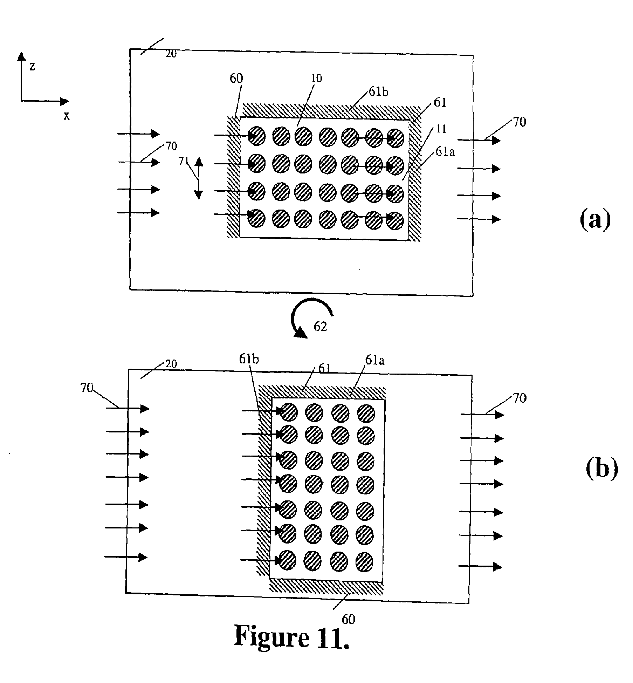

With N=8 (cfr. above calculation on sampling distance of 2.l=2 mm), and with a=200 $\mu$m, h=100 $\mu$m and d=0.1 $\mu$m, the gain in screening time now is of the order of 16,000 (still taking $\alpha$=1). As the presented calculation assumes a 1-D model, it has been neglected that the spots are only addressed row by row, whereas in a diffusion based-system the rows and columns are addressed simultaneously. However, turning the assay plate by 90° after having completed a given run and then repeating the assay (cfr. Fig. 11) only reduces the throughput by a factor of 2, still leaving sufficient room for improvement with respect to diffusion-driven screening systems.

In the above reasoning, it has also become obvious that, in the shear-driven method according to the present invention, the array only has to be contacted with a volume corresponding to that covering approximately 8 to 10 spot rows, whereas in a diffusion-based array a volume corresponding to 100 to 300 spot rows is applied (from which each spot only sees about 5 to 10 %). This fact implies that in the method according to the present invention, a ten or twenty fold reduction of the sample amount is foreseeable with respect to the amounts currently needed for a classical hybridisation assay.

Approaching the problem in another way, and considering to send the entire 10 to 200 $\mu$l sample volume typically used in conventional diffusion-driven micro-arrays in a shear-driven mode through a 100 nm thick channel, each spot would roughly 'see' 10 to 20 times more sample. It is hence obvious that, in this operation mode, the detection limits of the currently used diffusion-driven systems can be strongly surpassed.

Considering the above calculation on the required fluid velocity, it should be obvious that in the method according to the present invention, the fluid layer should preferably be transported at a velocity ranging between 1 mm/s (for slow-diffusing, long DNA strands) to about 40 cm/s (for more rapid diffusing, short DNA strands). It should be noted that the above described method according to the present invention may also be applied to accelerate the screening of other molecules, such as proteins or potential drug substances.

According to a preferred embodiment of the present invention, the spacing between the first and second surface in the device of the present invention is controlled by an array of micro-machined spacers, protruding from at least one of the surfaces, and preferably being coated with a wear-free and low-friction material layer.

An array of micro-machined spacers 50 is arranged on the moving second and/or the stationary first surface and allows to keep the distance between the moving and the stationary plate as constant as possible. These spacers may have any desired shape, including, but not limited to cylinders, truncated cones, semi-spheres, cubes and rectangular blocks. In one embodiment, the micro-machined channel spacers substantially extend along the entire stationary first surface length, with their longest dimension oriented along the x-direction. In this preferred embodiment, the channel spacers should preferentially have a substantially linear shape when the moving wall (second surface) is translated in a substantially rectilinear direction, whereas they should preferentially have a circularly curved shape when the moving wall is subjected to a rotational motion. The channel spacers may be made from, or coated with, any suitable wear-resistant material known to those skilled in the art of tribology. The channel spacers may be manufactured according to any of the suitable layer deposition method (e.g. CVD) and etching techniques (e.g. lift-off etching) known to those skilled in the art of micro-machining. Examples of processes for the machining of spacer structures having nanometrically small height tolerances, together with processes for the addition of a wear-free coating layer on top of these structures, can for example be found in Tanaka, H., *et al.,* 1993, *J. Tribol.* 115, 573-576; Li, Y. and Menon, A. K., 1995, *J. Tribol.* 117, 279-284. To keep the two surfaces in intimate contact, a load 501 may be applied.

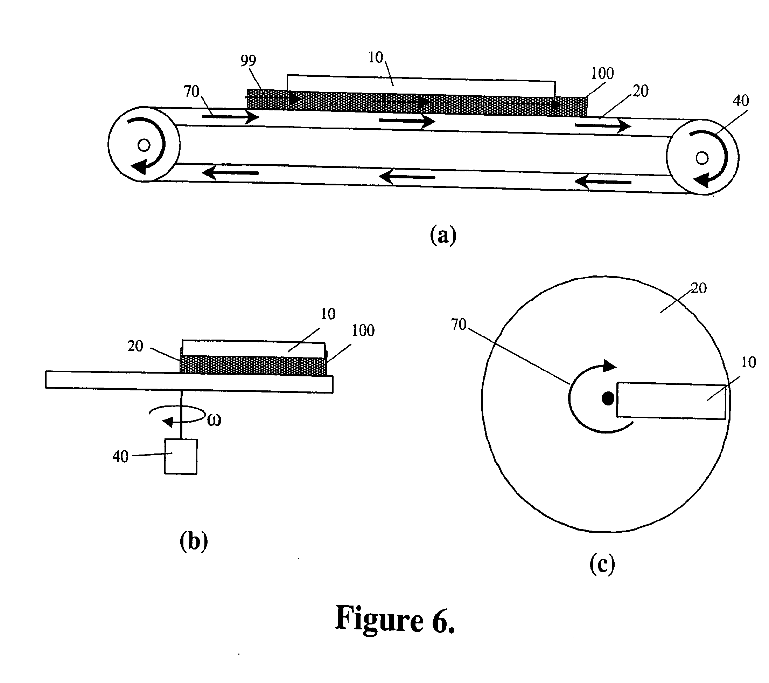

In one basic embodiment of the present invention, as is shown in Figure 4c, the moving second surface may be moved in a linear, once-through mode past the stationary first surface. However, in order to be able to transport a sufficient amount of sample, it is preferable to have a moving surface system which provides an infinite (i.e., continuously recirculating) flow path. Figure 6 shows conceptual drawings of moving belt (closed or not) and rotating disk-like devices providing such an infinite flow path.

According to yet another embodiment, said second surface iof the device of the present invention is a linearly movable flat plate, a linearly movable flexible belt, a rotatable device or a rotatable cylinder.

As explained before, said moving second surface is used to generate a flow past more than on of the target molecules tethered onto the surface of the substrate.

According to a preferred embodiment, said second surface in a device of the invention is a linearly movable flat surface, a linearly movable flexible belt, a rotatable disk or a rotatable cilinder.

The movement of the moving second surface may preferably be effected using any suitable motorized displacement device (not represented in drawing), including, but not limited to, a rotating disk table or a linear automatic translation system.)

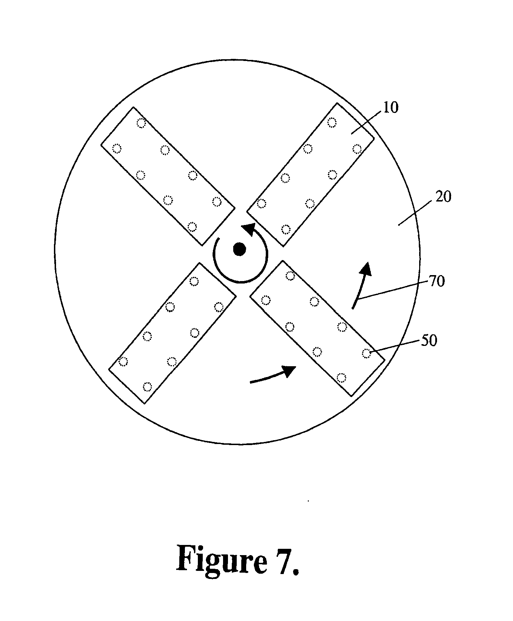

Figure 7 shows one of the many conceivable lay-outs of the device of the present invention in which a single moving surface 20 may be used to organize the sample transport past a multitude of array plates 10. In the drawing, the channel spacers 50 used to maintain a substantially constant distance between the moving and the stationary surfaces are represented as being partly visible through the stationary array plates 10. A similar multi-array system may be configured for the case of a moving belt device, e.g., by putting several different array plates behind each other.

According to yet another embodiment, the moving second surface of the device of the present invention carries an array of micro-machined protrusions extending from said second surface. Said micro-machined protrusions provide additional sustainment of the fluid motion, and/or can be used to induce additional mixing in the radial and lateral direction, and to limit the mixing in the axial direction.

According to another embodiment, the first surface comprises an array of porous holes.

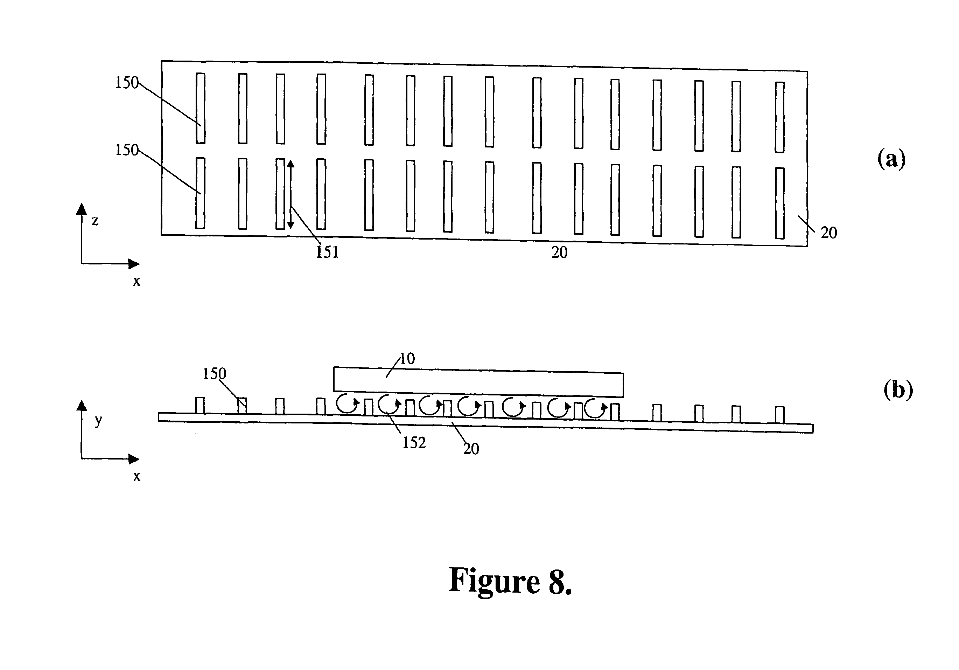

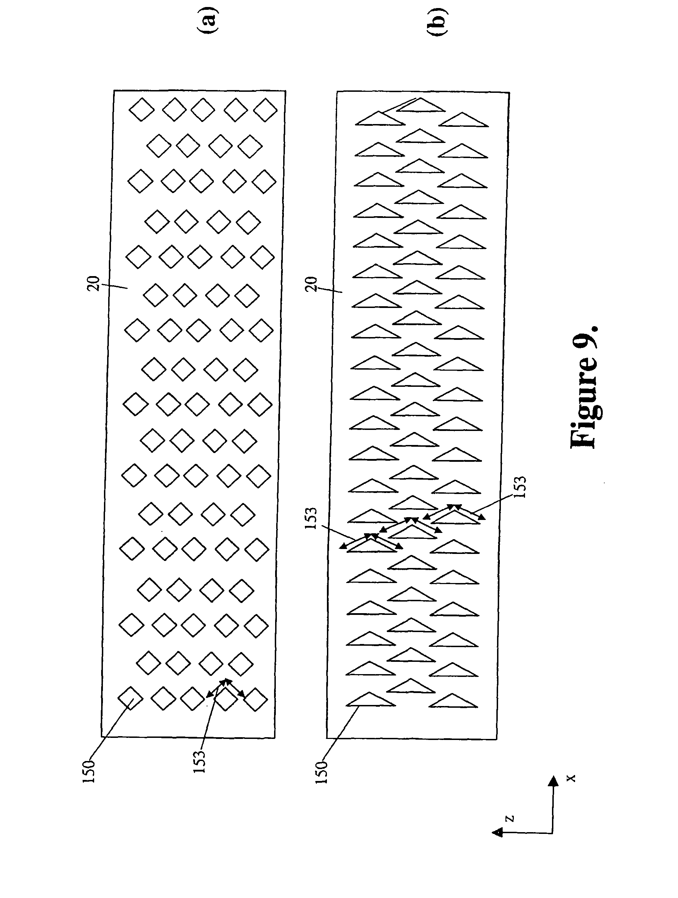

In one possible variant (Figure 8), said protrusions have the shape of rectangular rods, with their longest dimension 151 oriented along the z-direction and of course selected such that it fits between the channel spacers. Apart from providing an additional sustainment of the fluid motion (i.e., the mean fluid velocity will be larger than in the absence of the protrusions), said protrusions might also induce a desired additional radial mixing along direction 152. To create an optimal mixing pattern, the interval distance between the different protrusions in the x-direction should preferably be equal to between 1 and 10 times the channel height. It is yet another advantage of the embodiment represented in Figure 8 that the diffusional dispersion in the axial direction is reduced. Other preferred protruding elements to be arranged on the moving surface are depicted in Figure 9. In these embodiments, the shape and position of the protrusions 150 is selected such that they generate intermittent diversions 153 of the flow direction. Such diversions of the flow direction are highly preferred because they induce an additional flow in the z-direction, wherein the transport normally has to occur by the slow molecular diffusion. In an alternative approach, and without loosing their effect, the protrusions depicted in Figure 9 may also be arranged on the stationary first surface.

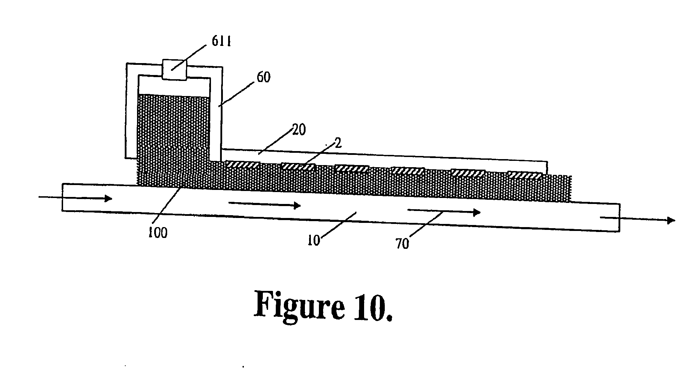

Figure 10 shows another preferred embodiment according to the present invention, wherein a so-called sample holder device 60 is attached to or integrated within the plate structure 20 carrying the array of receptor molecule spots 2. The sample holder device may for example serve as a means to prevent evaporation of the part of the sample 100 waiting to be transported through the screening channel formed by surfaces 10 and 20. When positioned at the channel outlet, the sample holder device may also serve as a means to collect the sample for subsequent analysis after having elapsed one passage through the screening channel. Preferably, the sample holder device should have a closeable connection 611 with the surrounding atmosphere or with a pressurized or vacuum chamber (not shown in drawing). The sample holder devices needed in the present invention may be manufactured with any standard micro-machining technique, such as reactive ion etching, LIGA and wet etching, and in one preferred embodiment, the sample holders are machined directly into the substrate carrying the receptor molecule spots. In another preferred variant, the sample holders are integrated within a vacuum chuck, or any other suitable fixation device, used to attach the stationary surface to a fixed holding frame (not represented in any of the drawings in the present document).

According to yet another embodiment, said second and/or first surface of the device of the present invention are sufficiently thin and flexible to conform optimally to the opposing surface.

In principle, the moving wall surface 20 may be made of any sufficiently flat and smoothly polished substrate made of any suitable material like glass, silicon, polymers,... ; and preferably between 50 and 1000 $\mu$m thick. To improve the functioning of the devices according to the present invention, the surface, or parts of the surface, of the moving second and/or the stationary first surface may be coated with a wall repellent layer such as for example a hydrophobic coating (to prevent the sample molecules from sticking or binding to the surface at undesired and non-specific positions). Such a coating can for example be a hydrophobic mono-layer of 3-[(Heptafluoroisopropoxy)-propyl]trichlorosilane. In another aspect, it is also preferred to provide a low-friction, low-wear-coating such as for example a Teflon layer (to improve the tribological behavior of the devices) on the parts of the second moving and/or the stationary first surface sliding past each other. The parts where such a coating is needed are the top surface of the micro-machined spacers 50, and the parts of the opposing surface coming into contact with said spacers.

According to yet another embodiment, said first surface of the device of the present invention is covered with a 1-D or 2-D array of receptor molecules.

The first surface may be covered with a 2-D array of substantially circular or square shaped receptor molecules, or either with a 1-D array of receptor molecule spots having one dimension much longer than the other, and substantially extending along the entire width or length or first surface.

According to yet another embodiment, the device of the present invention comprises means for temperature control and/or detection means, and/or means for the automated displacement of said second surface. The temperature in the devices according to the present method may for example be controlled using a micro-machined resistive circuit integrated on the back of the substrate carrying the stationary surface, or by simply contacting said substrate with a heating block. The fact that, due to the sub-micron channel thickness, the heat transfer in the channels is extremely rapid, is another advantage of the present invention.

In all the disclosed embodiments of the method according to the present invention, the detection of the amount of bound ligands or target analytes ma y occur by any of the detection methods known to those skilled in the art of micro-array and biosensor detection, including optical, electrical, acoustic or radioactivity-based detection methods. In one preferred embodiment, the detection occurs off-chip, after appropriate washing and drying, using array scanners such as for example the scanning confocal laser systems or the CCD-based detection systems which are currently available on a commercial basis. In another preferred embodiment, of especial use for the measurement of binding kinetics, the detection occurs on-chip.

In another aspect, the present invention also relates to a method for binding one or more target molecules comprised within a fluid sample to at least one receptor molecule at least partly tethered to a first surface of a substrate, wherein said target molecules are transported parallel to said first surface by means of a shear-force field.

The advantages of using said shear-force field have been discussed in full detail above in relation to the apparatus of this invention.

According to an embodiment, the shear-force field in the method of the invention is originating from a second surface being larger than said first surface and moved mechanically in a substantially parallel mode past said first surface.

According to yet another embodiment, the contact time between the target and receptor molecules in the method of the invention is taken sufficiently long, preferably between 0.1 ms and 10 minutes. Said contact time may be controlled accurately by controlling the velocity of the second moving surface. A contact time between 0.1 ms and 10 minutes should allow completion of the binding reactions to be investigated.

It is yet another advantage of the embodiments according to the present invention that it is very straightforward to switch from a continuous flow mode to a transport mode consisting of a continuous series of flow-stop sequences. This can readily be achieved using one of the commercially available automated linear or rotational displacement system, e.g. driven by a stepping motor, and offering a displacement resolution in the micrometer scale, as the driving means for the motion of the moving wall. It is well known that such displacement systems can easily be programmed to perform a series of alternating move-stop sequences, and that the displacement length and velocity, as well as the duration of the pauses, can be selected with a very large degree of freedom. The possibility to perform alternating flow-stop sequences is highly advantageous in cases where a strong convective flow disturbs the binding events: during the (short) stop periods, the binding process can take place undisturbed by any convection effects, while the flow periods are used to generate a rapid renewal of the fluid layer covering a given receptor molecule spot.

Preferably, this alternating flow-stop transport mode should be organized such that rapid fluid displacements (with a velocity preferably between 1cm/s and 1 m/s and covering a distance preferably equaling the distance between two consecutive spot centers) are alternated with stop-periods taken sufficiently long (for example between 1 ms and 1 s) to allow the matching target molecules to hybridize or bind to the receptor molecules in the absence of any convection or shear-force effects. In this way, a given sample liquid volume (with ground surface approximately equal to the surface of a given spot, and with a height equal to the channel height) is shifted from spot to spot in a step-like manner, hence traveling an entire DNA-chip surface in a relatively short time period and meeting a large number of different receptor molecule types. From the point of view of the receptor molecule spots, the displacement action is advantageous because the thin diffusion layer (say 100 nm thick) on top of the spots is continuously refreshed with fresh sample liquid. As this convective supply can be organized orders of magnitude more rapidly than the diffusive transport in the thicker fluid layers (say 50 micrometer thick) typical for conventional DNA-hybridisation, the gain in screening time can easily be understood.

To perform the binding screenings and assays according to the present invention, all the fluid composition (salt concentration, pH,..) variation methods, and all the temperature and electrical field variation methods known to those skilled in the art of DNA and protein micro-array screening may be used.

According to yet another embodiment, in the method of the invention said first surface is subjected to a translational or rotational motion in a direction different from the direction of motion of said second surface.

With an exception made for the embodiments in Fig. 9, the embodiments presented up to now have a common drawback in that a shear-flow, for example being generated in direction 70, only addresses the receptor molecule spots 2 in a row by row manner, whereas the transport in the perpendicular direction (direction 71) still has to occur by the slow molecular diffusion process (Figure 11a). One possible approach to solve this problem would be to recollect the sample in a sample holder device 61 extending along at least two side-walls of the assay plate 20, turning the assay plate 20 by 90°, according to direction 62, and repeating the screening process in direction 71. Due to the capillary force effect, liquid collected at the end 11 of the receptor spot surface 20 will automatically spread equally over both legs 61a and 61b of sample holder device 61.

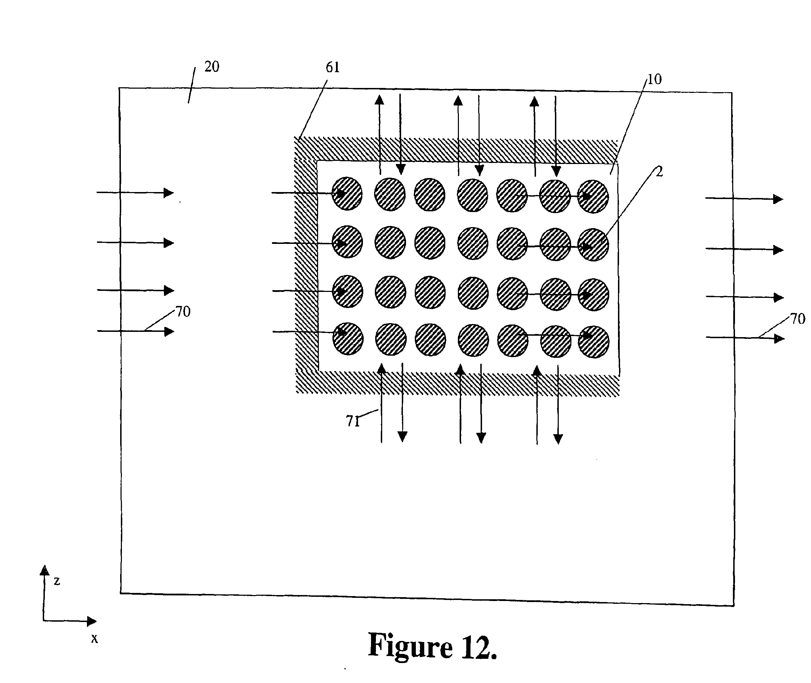

In another preferred embodiment of the present invention, a convective transport in two different directions 70 and 71 is generated as shown in Figure 12. While the basic movement in direction 70 is effected by moving surface 20, the surface 10 carrying the immobilized receptor molecule spots 2 may be subjected to a, preferably reciprocal motion in direction 71. To this end, a second motor device (not shown in the drawing) may be used. To have sufficient sample available to be transported along both the direction 70 and 71, a sample holding device 61 extending along at least three side-walls of the assay plate 10 should preferably be put in place, as indicated in Figure 12.

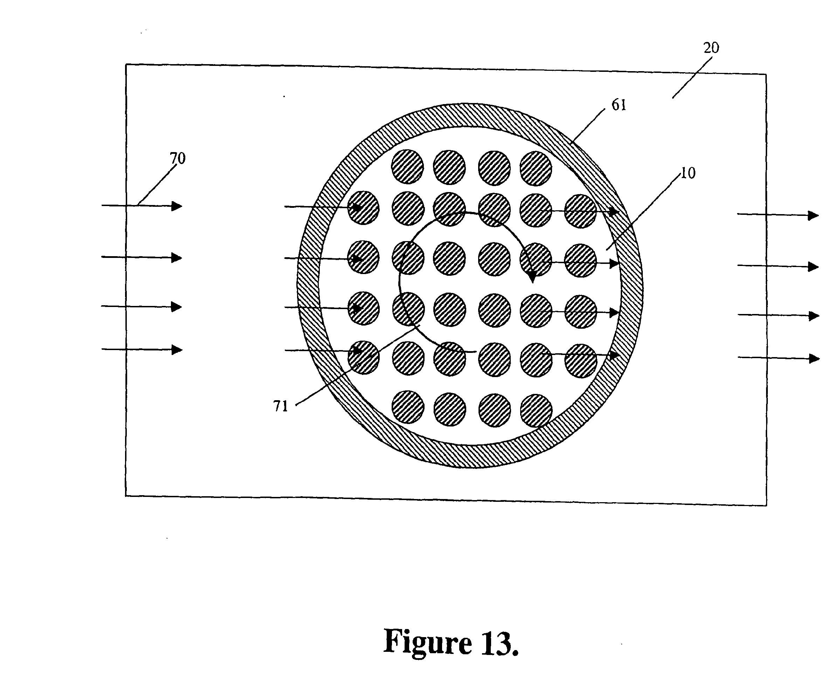

Yet another preferred embodiment of the present invention allowing to generate a convective transport in more than one direction is depicted in Figure 13. in this embodiment, the surface 10 carrying the receptor molecule spots 2 is subjected to a rotational movement 71. In this embodiment, a sample holding device 61 should preferably extend along the entire circumference of the assay surface 20.

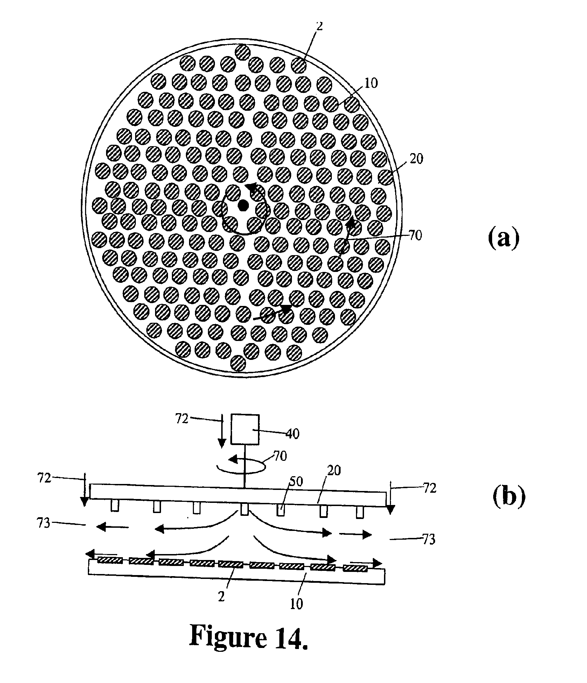

In another preferred embodiment (see Figure 14), both the stationary surface 10 carrying the receptor spots and the moving surface 20 have a disk-like shape. In this preferred embodiment, a sample holder device is no longer necessary, because the whole channel space between the two disk surfaces is used to hold the entire sample. The generation of a relative rotational movement between the two disks (it actually does not matter which one of the two surfaces is rotated) then allows to generate a convective transport in the tangential direction 70. This is highly advantageous because the convective transport over the entire disk circumference occurs on a time scale which is orders of magnitude smaller than the pure diffusive transport.

In a further variant of the dual rotating disk concept, the gap between the two disks is gradually decreased during the assay operation, by subjecting one of the two disks to a movement in direction 72. Preferably, an array of micro-machined spacers 50 is used to maintain a minimum spacing at the end of the operation. In this preferred embodiment, the movement of one of the two surfaces 10 or 20 in direction 72 induces a convective flow in the radial direction 71, with a net outward fluid motion through the peripherical disk region 73. This is a highly preferred situation, because it implies that a convective transport in both the tangential and the radial direction is established. Preferably, the initial spacing between the two disk surfaces should be between 5 to 100 $\mu$m, whereas the final spacing should preferably be between 50 to 1000 nm.

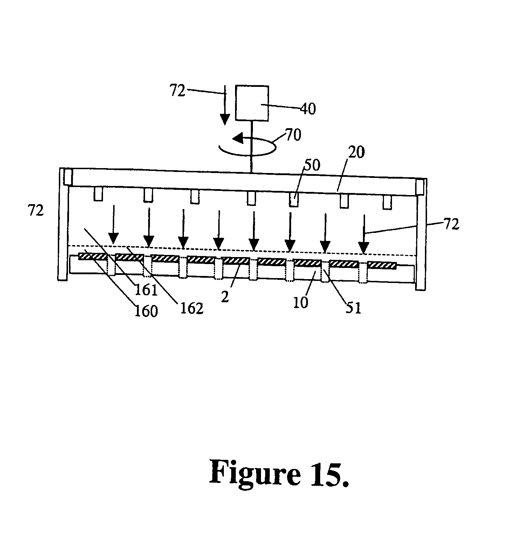

In an alternative version of the decreasing channel gap system presented in Figure 14, the outflow can also occur through an array of pores or micro-machined holes 51 etched through the receptor surface (Figure 15). The holes can be homogeneously distributed over the receptor surface, but they can also be concentrated at a specific region, e.g., near the disk center. The decreasing channel gap systems presented in Figure 14 and 15 allow to omit the sample holder devices in the embodiments presented in Figures 10-13: because of the small molecular diffusivity, the receptor molecules on the receptor surface only 'see' the sample molecules in a fluid layer 160 of only a few molecular diameters thick immediately adjacent to the receptor surface. The target molecules present in the fluid layers 161 farther away from the receptor surface are simply awaiting their turn before they are can enter the active zone 160 (driven by the fluid motion in direction 72). The layer 161 hence in fact acts as a sample holder. In Figure 15, the boundary between zones 160 and 161 is represented by the dashed line 162. In a preferred embodiment, the velocity of the rotation 70 is sufficiently larger than the fluid velocity in direction 72, such that the sample molecules present in layer 160 have had the time to be transported at least one time around the entire stationary receptor disk surface 10 (cfr. Figure 14a) before they leave the system through the holes 51.

According to yet another embodiment, the method of the present invention may be used for the purification of production quantities of a given target analyte comprised within a fluidic sample. Said method is realized by covering larger portions of the surface of the substrate with the same receptor molecules. After completion of the binding process, the second moving surface may be used to transport a given volume of desorbing fluidic sample past said receptor molecules to desorb and collect the bound target analytes.

Considering the possibility to transport large fluid volumes, the method according to the present invention may be used for the purification of production quantities of antibodies, growth factors, probe DNA, etc... In this case, larger portions of the receptor spot surface, preferably ranging between 10 to 100%, should be covered with the same receptor molecule type. After subsequent stringency testing, 'harvesting' of the selectively captured molecules may then occur by passing a desorption-promoting liquid solutions through the channel, by changing the temperature conditions, by applying an electrical field, or by applying any other desorption or regeneration method known to those skilled in the art of micro-array screening or bio-sensor measurements. It should again be noted here that, considering the achievable degree of channel thickness reduction, it is one of the advantages of the method according to the present invention that the desorbed molecules may be collected with a minimum of dilution, simply by using the moving wall to transport a given volume of desorbing liquid past the receptor molecules.

According to yet another embodiment, in the method of the present invention the amount of sample contacted with the receptor molecules preferably ranges from a few picoliter to a few hundred microliter.

Said amount may be exactly controlled by the distance over which the second surface is transported, and wherein, during the injection, preferably no other liquid is present upstream from the sample introduction point, and wherein the non-entered sample is aspirated away, wiped away, or flushed away.

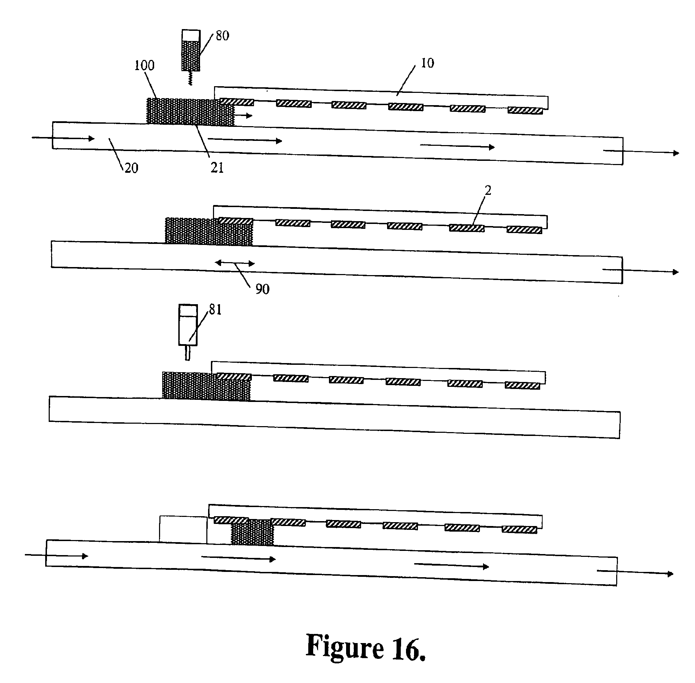

All embodiments thus far presented have been especially designed for the treatment of large sample volumes (i.e., for the case in which the sample volume is typically equal to or larger than the channel volume). When the samples are sufficiently concentrated, as is often the case for combinatorial chemistry library drug screening, it is desired to be able to treat smaller sample amounts. At this point, another advantage of the method according to the present invention arises. Following the injection procedure depicted in Figure 16, and with the possibility to control the displacement 90 of the moving wall 20 with even a sub-micrometer displacement accuracy (using for example an automatic linear displacement system with stepping motor), it is obvious that extremely short sample plugs 100 may be injected in a controllable and reproducible way. In Figure 16, it is shown how, using two different injection devices, one filled with sample (injection device 80) and one filled with a sample-free liquid solution (injection device 81), the sample injected with device 80 and which has not been entered into the channel may be diluted away using injection device 81. When for example using the piezo-electrically actuated and micromachined injection nozzles known to those skilled in the art, high speed dispensing of minute sample amounts is possible and very high injection frequencies may be obtained. Referring to.the sequence in Figure 16, it should be noted that the sample should preferably be added after removing (e.g., by wiping or aspirating) all liquid from the surface 21 in front of the channel inlet. In this preferred embodiment, no other liquid is present upstream from the sample introduction point, and hence dilution of the sample is avoided (cfr. the situation in Figure 10). Injection of the sample may also occur through a miniaturized sample holder devices as the one represented in Figure 10. Another advantage of the possibility to use the movement of the channel wall to control the injection volume in the method according to the present invention is that very small injection volumes (order picoliter) can be handled without causing any unnecessary dilution.

According to yet another embodiment, the second surface in the method of the invention may be used to inject and transport well-controlled volumes of fluid mixtures with desorption-promoting characteristics. This may be done for instance for stringency testing, for the desorption and the collection of the bound molecules, for the regeneration of the receptor molecules and for ligand displacement analysis.

In a manner similar to the injection and the transportation of sample liquids, the embodiments disclosed in this invention may also be used to inject and transport well-defined plugs or volumes of sample-free desorption-promoting liquid solutions, for example to perform binding stringency tests, for the removal of non-specifically bound molecules, or to desorb the bound ligands from the receptor molecules.

A sample holder device similar to the one depicted in Figure 10 can also be used to hold a stringency testing liquid or washing liquid prior and or after its passage through the channel. In a preferred embodiment, the sample holder device is used as a pre-mixing chamber wherein the composition of the stringency testing liquid is continuously varied, such that a gradient-like stringency test can be performed.

According to yet another embodiment, in the method of the present invention a limited sample plug containing one or more different target molecules is injected into the channel formed by said two surfaces, and wherein said sample plug is subsequently transported past at least one of the tethered receptor molecule spots, and wherein the axial width of said sample plug preferably ranges between 0.5 and 10 times the width of said spots and wherein said sample plug is preceded and followed by a sample-free liquid plug having and axial width which is preferably larger than 100 $\mu$m.

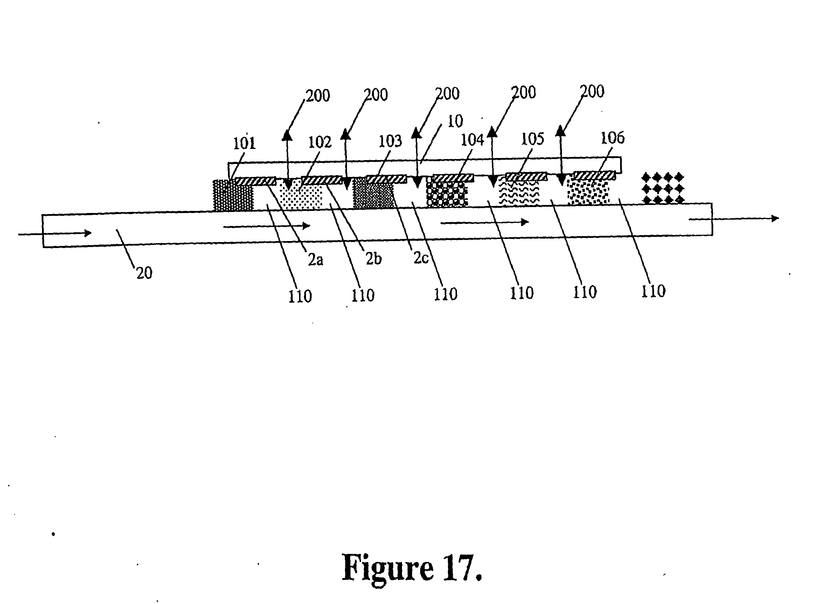

Combining the possibility to inject sharply delimited sample plugs with the fact that the degree of axial dispersion in channels with a sub-micron thickness is very small, it is obvious that in one of the preferred methods according to the present invention, very short plugs (i.e., with a length of the order of 10 to 500 $\mu$m) of different samples 101-106, only separated by comparably short plugs 110 of sample-free buffer liquid (i.e., such that the sample plugs are separated by sample-free liquid plugs with a length of only 50 to 500 $\mu$m), may be transported through the micro-array channel formed by surfaces 10 and 20 without any significant hydrodynamic intermixing or carry-over between the different sample plugs (Figure 17). The possibility to generate high frequency transports of short sample plugs may be understood from the fact that the degree of axial dispersion or peak broadening in channels with a sub-micron thickness is extremely small, because any difference in radial velocity is immediately wiped out by the rapid molecular diffusion. This is a fact which is known for pressure-driven flows, but which may readily be transposed to shear-driven flows. An expression for the axial dispersion in pressure-driven laminar circular pipe flow is given in Cussler, E. L., 1984, Diffusion: mass transfer in fluid systems, Cambridge University press, Cambridge, USA, pp. 90.

Injecting different short samples pulses consecutively one after the other and transporting them across an array of different receptor molecule spots, the currently described embodiment enables true high throughput screening of highly concentrated samples. In a preferred variant, an array-format detecting device (such as a Charge Coupled Device-array, or a micro-lense Laser Induced Fluorescence array of the type presented in Bruno, A. E., *et al.,* 1998, Micro-optical fluorescence detection for chip-based multiplexed analysis systems, in Micro Total Analysis Systems, Eds. Harrison, D. J. and van den Berg, A., Kluwer Academic Publishers, Dordrecht, The Netherlands, pp.281-285) capable of monitoring the entire array plate is used to detect and monitor the occurrence of binding events at the receptor molecule spots. In Figure 17, the double-sided arrows 200 represent possible detection points for such an array-detection system.

According to yet another preferred embodiment, the method of the present invention may be used for determining the binding kinetics and the binding equilibrium constant by varying the time during which said limited sample plug is in contact with said receptor molecule spots and by measuring the amount of selectively bound fluid target molecules with an on-spot detection device during or after the passage of the sample.

According to yet another more preferred embodiment, said limited sample plug in the method of the invention has a width preferably ranging between 0.5 and 0.8 times the width of said receptor molecule spots, and wherein the movement of the second surface is stopped when said sample plug has reached the position of a given receptor molecule spot.

According to yet another more preferred embodiment, binding of said target molecules in the sample with said receptor molecules on the surface of the substrate is followed by a chemical reaction step, and wherein the reaction kinetics are determined by varying the time during which said sample plug is in contact with said receptor molecule.

According to yet another more preferred embodiment, in the method of the present invention at least one detection device is positioned at a given distance, preferably between 100 and 1000 $\mu$m downstream or upstream of a given receptor molecule, and wherein the difference in target analyte concentration between and after the passage of said sample plug at said receptor molecule is used to determine the amount of bound target analytes.

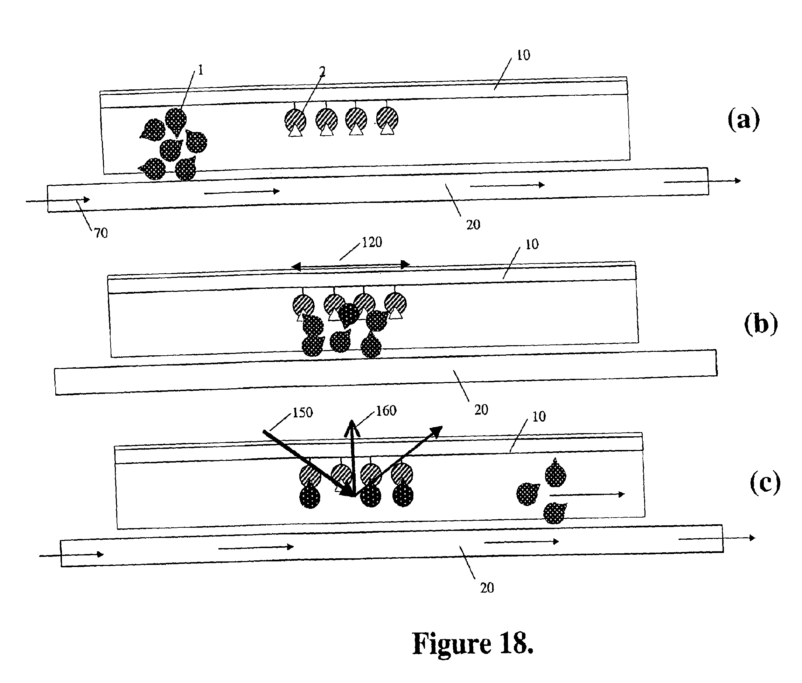

The schematic representation given in Figure 18 shows how, by injecting a sharply delimited plug of sample molecules 1, the method according to the present invention may also be used to determine the intrinsic kinetics of target-receptor binding events. Exploiting the possibility to use microfluidic channels with a thickness which is only a limited number of times larger than the molecular diameter of the sample molecules, the molecules only have to diffuse over a negligible distance, such that the recorded binding time is only related to the actual binding process, and is not biased by the slow diffusion process. A preferred binding kinetics measurement method is based on the injection of a sample plug which is preferentially of about the same width as the width 120 of the receptor molecule spot 2, and comprises a first step in which said sample plug 100 is transported by means of a shear-driven flow towards said receptor molecule spot 2 (Fig. 18a). When the sample plug reaches said spot (Fig. 18b), the flow may either be stopped during a given pre-selected contact time $t_{cont}$ or may be continued at a given pre-selected velocity u selected such that the time of spot passage or dwell time $t_{dwell}$, determined by the ratio of the spot length 120 and the selected velocity u, equals the contact time $t_{cont}$ of interest. This contact time preferably ranges between 0.5 milliseconds and several hours, but will preferably be between 1 millisecond and 10 minutes. The ultra-short contact times in the order of 1 millisecond may be achieved for instance by passing a fluid with a velocity of 0.2 m/s past a spot with an axial width of 20 $\mu$m. At the molecular level, the contact times are even orders of magnitude smaller. It should hence be obvious that the method according to the present invention allows to realize ultra-short molecule to molecule contact times which are of the order of the intrinsic binding times. This is a very attractive advantage of the method according to the present invention. Using one of the commercially available high resolution automated displacement systems equipped with a stepping motor and providing even sub-micrometer displacement accuracy and allowing for large displacement velocities of the order of 10 cm/s or more, both the position of the sample plug and the displacement velocity may be accurately controlled.

By performing different experiments at different contact times, and plotting the amount bound N versus the contact or dwell time $t_{dell}$, the full kinetic binding curve may be established. To determine the rate of the desorption reaction, a plug of a liquid solution with desoprtion-promoting characteristics may be injected and transported a given distance behind the sample plug.

To increase the detection limit of the binding kinetics measurement according to the method of the present invention, it is also possible to inject larger sample volumes (i.e., with a longer plug length) and subsequently transporting them at a predefined velocity past the receptor molecule spot. Multiplying the total amount $N_{tot}$ bound under these conditions by the ratio of the dwell time ($t_{dwell}$=spot length/fluid velocity) to the total plug contact time ($t_{total}$=sample plug length/fluid velocity), a quantity N is obtained which contains the same kinetic information as the quantity N obtained from the short plug experiments cited above, but which has a smaller measurement error.

According to yet another embodiment, in the method of the present invention said moving second surface is used to transport a plug of desorbed target molecules or the products of an enzymatic assay reaction in a single plug towards a detection device. Said detection device may be for instance a mass spectrometer, capable of quantifying and identifying the desorbed target molecules or the enzymatic reaction products, and located downstream of the receptor molecule.

According to yet another embodiment, in the method of the present invention a stringency test is performed by applying a sudden change in fluid composition or temperature, or by applying an external electrical field.

According to yet another embodiment, the method of the present invention may be used for measuring the amount of selectively bound target molecules to receptor molecules tethered onto the surface of the substrate, using a detection means. The amount of selectively bound target molecules may be measured after appropriate washing and drying of said surface, using for example a scanning confocal laser microscope, a CCD-array or a scanning MALDI/MS device.

According to yet another embodiment, the amount of selectively bound target molecules may be measured on-chip. This may be done using for example total internal reflection fluorescence, surface acoustic wave analysis, surface plasmon resonance, electrochemical analysis, or electrical impedance, conductance, amperometric, or capacitance analysis to distinguish between the bound and unbound molecules.

Currently, a large number of methods allowing to continuously monitor the amount of molecules bound at the spot surface without being biased by the unbound target molecules present in the fluid phase surrounding the receptor molecules, are known. As is known to those skilled in the art, such methods include, but are not limited to, surface plasmon resonance, surface acoustic wave, total internal reflection fluorescence, electrochemical methods, or potentiometric, conductometric, amperometric and capacitance methods. In yet another preferred embodiment, the detection of the amount of bound molecules is performed after the sample plug has been transported away from the binding spot (cfr. Fig. 18c) and has been replaced by a sample-free liquid solution having the right salt and buffer composition to prevent desorption of the bound target molecules. In this way, the method of detection should not be limited to surface detection techniques such as the methods cited above, but more universal and cheaper detection methods such as Laser Induced Fluorescence (LIF) may be used. As depicted in Figure 18c, this method is based on the use of an incidenting laser beam 150 with coherent light of a given wavelength to induce the emission of a fluorescent electro-magnetic field 160 at another wavelength, which may then be collected under a different angle. In yet another preferred embodiment, the amount of bound molecules is detected using a detection device located at a given (short) distance downstream or upstream of a given receptor molecule spot. In this embodiment, the decrease in target analyte concentration is used as an indirect measure for the amount bound. In a preferred embodiment, the amount of free target molecules may be determined both before and after the contact with the receptor molecule spot using a single detection point. Positioning the detection device at a given position downstream of the receptor molecule spot, said detection device may in a first transport phase be used to measure the total amount of unbound target molecules present in a given sample before the sample has been in contact with the receptor molecule spot. After the sample has passed the detector and has been brought into contact with said receptor molecule spot, the moving surface may be moved in the direction opposite to that in the first transport phase, such that the plug passes the same detection device again, allowing to determine the reduction of the free target molecule concentration.

In the embodiments where the bound molecules have to be desorbed and subsequently transported in a minimally diluted plug towards a detection device or towards the channel end (for fraction collection purposes), it is preferred that the velocity lines of the shear-driven flow are running parallel, such that a minimum of lateral intermixing (i.e., in the z-direction) occurs between the different sample plugs being analysed in parallel.

In another preferred embodiment according to the present invention, particularly suited to determine the structure and the composition of unknown ligands bound to the receptor molecules, the possibility to transport liquid plugs with a limited amount of axial dispersion may be used to transport a plug of a desorption-promoting liquid to a given receptor molecule spot, where the bound target molecules are desorbed and subsequently transported in a single concentrated plug towards a detection device, such as a mass spectrometer, located downstream of the receptor molecule spot. In the case of enzymatic reaction assays, the method according to the present invention may also be used to transport the reaction products in a highly concentrated plugs of towards a mass spectrometer device for subsequent analysis of the reaction product. In yet another embodiment, the characterization of unknown bound ligands may occur via matrix-assisted laser desorptionIonization mass spectrometry (MALDI/MS), after appropriate washing and drying of the receptor spot surface.

According to yet another embodiment, in the method according to the invention the measurements may be performed in a 1-D or 2-D array format and wherein a 1-D or 2-D detector array is used, and wherein all flow streamlines run in a substantially parallel mode to prevent intermixing between parallel flow lanes.

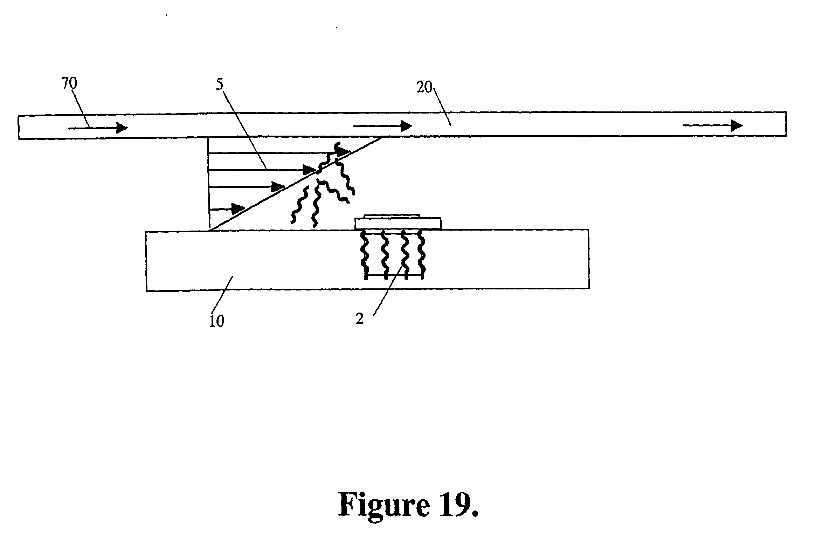

Most of the on-chip detection methods cited above, may also be applied in an array-format, wherein a multitude of spots is monitored simultaneously. Array-LIF detection using a 2-D array of micro-lenses has for example been demonstrated by Bruno et al. (1998). All electrical and piezo-electric and acoustic methods, may, using the wide range of currently existing micro-machining techniques, also easily be applied in an array format. Even multiplexed mass-spectrometry, using e.g., one MS to monitor the content of a 96-well plate, has recently been demonstrated (Foret, F. *et al.*, Microdevices for Electrospray Mass Spectrometry in Micro Total Analysis Systems, 1998 Eds. Harrison, D. J. and van den Berg, A., Kluwer Academic Publishers, Dordrecht, The Netherlands, pp.35-38). According to yet another embodiment, in the method of the invention different fluid substances may be transported in a single plug, i.e. at the same velocity, to investigate binding or reaction events involving a co-factor, or to perform competitive binding assays. In another embodiment (cfr. Figure 19), suited for applications requiring a large fluid velocity, the receptor molecules are immobilized in shallow etched recessions to protect them from the high shear-field 5.

In addition, it should be remarked that, in all the presented embodiments, the surface which is normally intended to generate the shear-flow might also carry selective receptor elements.

**Claims**

1. A device for the conduction of target-receptor binding reactions within a flow channel provided with receptor molecules, whereby said binding reaction is being generated upon contact of a fluidic sample comprising one or more target molecules with said receptor molecules, said device further comprises:

   a. first solid surface provided with discrete and isolated locations for such a binding reaction within said flow channel, and
   b. second surface is able to be moved in a parallel mode past or over the first surface,

   and wherein the channel space between the two surfaces is arranged to hold the entire sample.

2. A device according to claim 1 configured such that one surface rotates relative to the other surface.

3. A device according to claims 1 or 2 wherein both surfaces have a disk-like shape.

4. A device according to any of claims 1 to 3 wherein said second surface is configured to move in a continuous mode past or over the first surface.

**5.** A device according to any of claims 1 to 3 wherein said second surface is configured to move in an alternating series of move-stop sequences.

**6.** A device according to 5 wherein said move-stop sequence comprises a fluid displacement covering a distance equalling the distance between two consecutive binding reaction locations.

**7.** A device according to any of claims 5 or 6 wherein said move-stop sequence comprises a stop period of length to allow target molecules to hybridise or bind to the receptor molecules in the absence of convection of other shear force effects.

**8.** A device according to any of claims 1 to 7, whereby the second surface is larger than said first surface.

**9.** A device according to any of claims 1 to 7, **characterized in that**, the first surface comprises surface recessions having preferably about the same depth as the height of the receptor molecules immobilised therein.

**10.** A device according to any of claims 1 to 9, wherein the spacing between the first and second surface is controlled by an array of spacer means, protruding from at least one of the surfaces, and preferably being coated with a wear-free and low-friction layer.

**11.** A device according to any of claims 1 to 10, wherein the second surface carries an array of protrusions extending from said surface.

**12.** A device according to any of claims 1 to 11, wherein said first and second surface is sufficiently thin and flexible to conform optimally to the opposing surface.

**13.** A device according to any of claims 1 to 12, wherein said first surface is covered homogeneously with the same type of receptor molecules, or is covered with a one or two-dimensional array of different receptor molecule spots

**14.** A device according to any of claims 1 to 13, further comprising means for temperature control and/or detection means, and/or means for the automated displacement of said second surface.

**15.** A device according to any of the claims 1 to 14, wherein said first surface comprises an array of porous holes.

**16.** A device according to any of the claims 1 to 15 wherein said second surface comprises selective receptor means.

**17.** A device according to any of the claims 5 to 16, wherein said move stop sequences of the second surface are provided by a programmable automatic displacement system.

**18.** A method for binding one or more target molecules comprised within a fluid sample to at least one receptor molecule at least partly tethered to a first solid surface in a flow channel, wherein said target molecules are transported parallel to said first surface by means of a second surface, which second solid surface is moved in a parallel mode past or over said first surface, wherein the channel between the surfaces is used to hold the entire sample.

**19.** A method according to claim 18 wherein one surface rotates relative to the other surface.

**20.** A method according to claims 18 or 19 wherein both surfaces have a disk-like shape.

**21.** A method according to any of claims 18 to 20 wherein said second surface moves in a continuous mode past or over the second surface.

**22.** A method according to claims 18 to 21 wherein said second surface moves in an alternating series of move-stop sequences.

**23.** A method according to claim 22 wherein said move-stop sequence comprises a stop period of length to allow target molecules to hybridise or bind to the receptor molecules in the absence of convection of other shear force effects.

**24.** A method according to claims 22 or 23 wherein said first surface is subjected to a translational or rotational motion in a direction different from the direction of motion of said second surface.

**25.** A method according to any of claims 18 to 24 wherein a stringency test is performed by applying a sudden change in fluid composition or temperature, or by applying an external electrical field.

**26.** A method according to any of claims 18 to 25 used for measuring the amount of target molecules selectively bound to receptor molecules at least partly tethered to the first surface of the substrate.

**27.** A method according to any of claims 18 to 26 wherein the amount of selectively bound target molecules is measured on-chip.

**28.** A method according to any of claims 18 to 27 used for the purification of production quantities of a given target analyte comprised within a fluidic sample.

**29.** A method according to any of claims 18 to 28 wherein the amount of sample contacted with the receptor molecules preferably ranges from a few picoliter to a few hundred microliter.

**30.** A method according to any of claims 18 to 29 wherein the second surface is used to inject and transport well-controlled volumes of fluid mixtures with desorption-promoting characteristics for stringency testing.

**31.** A method according to any of claims 18 to 30, wherein the distance between said surfaces is gradually decreased during the course of the assay operation.

Figure 1.

Figure 2.

**Figure 3.**

**Figure 4.**

**Figure 5.**

EP 1 627 686 A1

EP 1 627 686 A1

**(a)**

**(b)**

**(c)**

# Figure 6.

**Figure 7.**

**Figure 8.**

EP 1 627 686 A1

EP 1 627 686 A1

Figure 9.

26

Figure 10.

EP 1 627 686 A1

**(a)**

**(b)**

**Figure 11.**

Figure 12.

EP 1 627 686 A1

Figure 13.

Figure 14.

Figure 15.

Figure 16.

Figure 17.

EP 1 627 686 A1

Figure 18.

Figure 19.

**European Patent Office**

## EUROPEAN SEARCH REPORT

Application Number

EP 05 02 4473

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 98/55858 A (DESMET GERT ;BARON GINO (BE); UNIV BRUXELLES (BE)) 10 December 1998 (1998-12-10) * line 27 - page 3, line 15; figures * ----- | 1-4,8-16 | B01L3/00 G01N30/92 G01N30/38 |
| X | EP 0 670 489 A (CIBA-GEIGY AG) 6 September 1995 (1995-09-06) | 1-4, 8-16, 18-21, 28-31 | |
| Y | * page 2, lines 4-11; figures * * page 3, line 44 - page 4, line 18 * ----- | 25-27 | |
| Y,D | US 6 017 696 A (HELLER ET AL) 25 January 2000 (2000-01-25) * the whole document * ----- | 25-27 | |
| A,D | WO 99/61896 A (JENNISSEN HERBERT PETER ;ZUMBRINK THOMAS (DE)) 2 December 1999 (1999-12-02) * the whole document * * page 14, lines 11-35 * * page 18, lines 1-18; figures * ----- | 1-31 | TECHNICAL FIELDS SEARCHED (IPC) B01L G01N B01D |

The present search report has been drawn up for all claims

| Place of search | Date of completion of the search | Examiner |
|---|---|---|
| Munich | 23 December 2005 | Persichini, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

**ANNEX TO THE EUROPEAN SEARCH REPORT
ON EUROPEAN PATENT APPLICATION NO.**                    EP 05 02 4473

This annex lists the patent family members relating to the patent documents cited in  the above-mentioned European search report.
The members are as contained in the European Patent Office EDP file on
The European Patent Office is in no way liable for these particulars which are merely  given for the purpose of information.

23-12-2005

| Patent document cited in search report | | Publication date | Patent family member(s) | | Publication date |
|---|---|---|---|---|---|
| WO 9855858 | A | 10-12-1998 | AT | 263370 T | 15-04-2004 |
| | | | AU | 8110598 A | 21-12-1998 |
| | | | DE | 69822820 D1 | 06-05-2004 |
| | | | DE | 69822820 T2 | 17-03-2005 |
| | | | DK | 986749 T3 | 02-08-2004 |
| | | | EP | 0882982 A1 | 09-12-1998 |
| | | | ES | 2218837 T3 | 16-11-2004 |
| | | | JP | 2002508840 T | 19-03-2002 |
| | | | PT | 986749 T | 30-07-2004 |
| | | | US | 6565752 B1 | 20-05-2003 |
| EP 0670489 | A | 06-09-1995 | JP | 8105873 A | 23-04-1996 |
| | | | US | 5595650 A | 21-01-1997 |
| US 6017696 | A | 25-01-2000 | AT | 211259 T | 15-01-2002 |
| | | | AU | 692800 B2 | 18-06-1998 |
| | | | AU | 8125794 A | 23-05-1995 |
| | | | BR | 9407952 A | 26-11-1996 |
| | | | CA | 2175483 A1 | 11-05-1995 |
| | | | CN | 1141078 A | 22-01-1997 |
| | | | CN | 1558207 A | 29-12-2004 |
| | | | DE | 69429535 D1 | 31-01-2002 |
| | | | DE | 69429535 T2 | 22-08-2002 |
| | | | DK | 727045 T3 | 15-04-2002 |
| | | | EP | 0727045 A1 | 21-08-1996 |
| | | | ES | 2170133 T3 | 01-08-2002 |
| | | | FI | 961843 A | 20-06-1996 |
| | | | HK | 1011079 A1 | 07-06-2002 |
| | | | JP | 9504910 T | 13-05-1997 |
| | | | NZ | 275962 A | 28-07-1998 |
| | | | NZ | 330036 A | 28-01-2000 |
| | | | NZ | 330037 A | 28-01-2000 |
| | | | PT | 727045 T | 28-06-2002 |
| | | | WO | 9512808 A1 | 11-05-1995 |
| | | | US | 5605662 A | 25-02-1997 |
| | | | US | 5929208 A | 27-07-1999 |
| WO 9961896 | A | 02-12-1999 | AT | 301831 T | 15-08-2005 |
| | | | AU | 760704 B2 | 22-05-2003 |
| | | | AU | 5149499 A | 13-12-1999 |
| | | | CA | 2333391 A1 | 02-12-1999 |
| | | | DE | 19980961 D2 | 03-01-2002 |
| | | | EP | 1082601 A1 | 14-03-2001 |
| | | | JP | 2002516993 T | 11-06-2002 |
| | | | US | 6843963 B1 | 18-01-2005 |

EPO FORM P0459

For more details about this annex : see Official Journal of the European Patent Office, No. 12/82